# EUROPEAN PATENT APPLICATION

(11) **EP 1 813 193 A2**
(43) Date of publication of application: **01.08.2007**
(21) Application number: 07001368.5
(22) Date of filing: 23.01.2007
(51) Int. Cl.: A61B 8/08, G01S 15/89

(54) **Apparatus and method for displaying an ultrasound image**

(30) Priority: 26.01.2006 KR 20060008122
(71) Applicant: MEDISON CO., LTD., Kangwon-do 250-870 (KR)
(72) Inventor: Hyun, Dong Gyu Discusser & Medison Building, Seoul 135-280 (KR)
(74) Representative: Schmid, Wolfgang

(57) **Abstract**

The method of displaying an ultrasound image, comprises: a) setting a probe on a predetermined portion of a target object including a moving object to transmit ultrasound signals to a specific slice of the moving object; b) forming and storing a plurality of sequential 2-dimensional ultrasound images based on ultrasound echo signals reflected from the specific slice, each of said sequential 2-dimensional ultrasound images having information of forming time; c) selecting n numbers of 2-dimensional ultrasound images in an order of forming time; d) superposing the n number of 2-dimensional ultrasound images in a time axis to thereby form a 3-dimensional ultrasound image; e) setting a moving cycle of the moving object and a plurality of markers representing a moving cycle interval on the 3-dimensional ultrasound image; f) displaying the 3-dimensional ultrasound image with the moving cycle markers; g) removing a first 2-dimensional ultrasound image from the n numbers of 2-dimensional ultrasound images; h) selecting and superposing a (n+1)^{th} 2-dimensional ultrasound image; and i) repeating the steps e) to h).

## Description

The present application claims priority from Korean Patent Application No. 10-2006-008122 filed on January 26, 2006, the entire subject matter of which is incorporated herein by reference.

### BACKGROUND

### 1. Field

The present invention generally relates to ultrasound image processing, and more particularly to an apparatus and method for displaying a 3-dimensional ultrasound image formed based on 2-dimensional ultrasound images.

### 2. Background

An ultrasound diagnostic system has become an important and popular diagnostic tool since it has a wide range of applications. Specifically, due to its non-invasive and non-destructive nature, the ultrasound diagnostic system has been extensively used in the medical profession. Modern high-performance ultrasound diagnostic systems and techniques are commonly used to produce two or three-dimensional diagnostic images of internal features of an object (e.g., human organs).

The ultrasound diagnostic system generally uses a wide bandwidth transducer to transmit and receive ultrasound signals. The ultrasound diagnostic system forms images of human internal tissues by electrically exciting an acoustic transducer element or an array of acoustic transducer elements to generate ultrasound signals that travel into the body. The ultrasound signals produce ultrasound echo signals since they are reflected from body tissues, which appear as discontinuities to the propagating ultrasound signals. Various ultrasound echo signals return to the transducer element and are converted into electrical signals, which are amplified and processed to produce ultrasound data for an image of the tissues. The ultrasound diagnostic system is very important in the medical field since it provides physicians with real-time and high-resolution images of human internal features without the need for invasive observation techniques such as surgery.

Generally, the ultrasound diagnostic system obtains raw 3D data (e.g., data on a coordinate system (x, y and z)) through a 3D probe by stacking consecutive frames. It then processes the consecutive frames by using a 3D rendering technique, thereby producing 3D static images. By using the static 3D images for ultrasound diagnostic purposes, one may easily and accurately observe, diagnose and treat the internal state of a human body without performing any complicated invasive procedures. Thus, the static 3D images are widely used. However, the static 3D images are not useful in observing a moving target object in real time, such as a fetus in the uterus.

In order to overcome this shortcoming, a live 3D imaging method and apparatus for providing a live 3D moving image (rather than static 3D images) have been developed. The live 3D image consists of fewer frames compared to a real-time 3D moving image. Thus, the live 3D image cannot completely represent the movement of a moving target object. However, since the live 3D image consists of more frames than static 3D images (e.g., 2 to 4 frames per second), it can represent the movement of a moving target object more smoothly than the static 3D images.

However, there is a limitation in completely scanning the movement of a rapidly compressing and relaxing object such as a heart by using a 3D ultrasound probe. Therefore, it is difficult to observe the movements of a heart and its valves, blood flow and the like in real time.

### BRIEF DESCRIPTION OF THE DRAWINGS

Arrangements and embodiments may be described in detail with reference to the following drawings in which like reference numerals refer to like elements and wherein:

FIG. 1 is a schematic block diagram illustrating an ultrasound diagnostic device constructed in accordance with one embodiment of the present invention;

FIG. 2 is a schematic diagram illustrating an example of setting a cycle of an electrocardiogram signal;

FIG. 3 is a flowchart illustrating a method of displaying an ultrasound image in accordance with one embodiment of the present invention;

FIG. 4 is a schematic diagram illustrating a plurality of 2-dimensional ultrasound images;

FIG. 5 is a schematic diagram illustrating a predetermined number of 2-dimensional ultrasound images for forming a 3-dimensional ultrasound image;

FIG. 6 is a schematic diagram illustrating a 3-dimensional ultrasound image formed by superposing a predetermined number of 2-dimensional ultrasound images in accordance with one embodiment of the present invention;

FIG. 7 is a schematic diagram illustrating a 3-dimensional ultrasound image on which a plurality of ECG cycle markers are set;

FIG. 8 is a schematic diagram illustrating an updated 3-dimensional ultrasound image; and

FIG. 9 is a schematic diagram illustrating an example of selecting two volumes in the 3-dimensional ultrasound image in accordance with one embodiment of the present invention.

### DETAILED DESCRIPTION

A detailed description may be provided with reference to the accompanying drawings. One of ordinary skill in the art may realize that the following description is illustrative only and is not in any way limiting. Other embodiments of the present invention may readily suggest themselves to such skilled persons having the benefit of this disclosure.

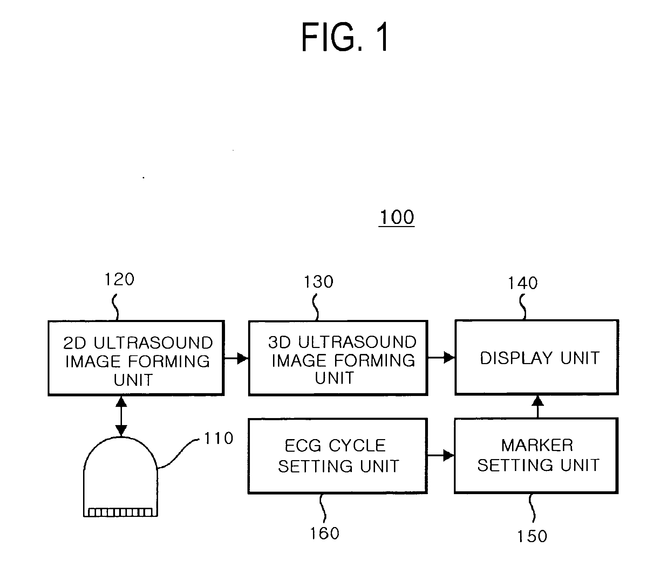

FIG. 1 is a block diagram illustrating an ultrasound diagnostic device, which is constructed in accordance with the present invention. As shown in FIG. 1, the ultrasound diagnostic device 100 includes a probe 110, a 2-dimensional ultrasound image forming unit 120, a 3-dimensional ultrasound image forming unit 130, a display unit 140, a marker setting unit 150 and an electrocardiogram (ECG) signal cycle setting unit 160. The probe 110 transmits ultrasound signals to a predetermined region of a target object, which includes a periodically moving object, and receives ultrasound echo signals. The periodically moving object may be a heart, a blood vessel or the like. The probe 110 may be any type of probe, which is capable of acquiring a 2-dimensional ultrasound image.

The 2-dimensional ultrasound image forming unit 120 forms a plurality of 2-dimensional ultrasound images based on the ultrasound echo signals according to time. The 2-dimensional ultrasound images may be stored in a memory (not denoted). The 2-dimensional ultrasound images are stored with forming time information. Hereinafter, the "forming time" may refer to the time at which a 2-dimensional ultrasound image is formed in the 2-dimensional ultrasound image forming unit 120. The 2-dimensional ultrasound images may be B-mode images, Doppler images or color-mode images.

The 3-dimensional ultrasound image forming unit 130 forms a 3-dimensional ultrasound image based on the multiple 2-dimensional ultrasound images. The 3-dimensional ultrasound image forming unit 130 selects a predetermined number of 2-dimensional ultrasound images and may store the selected 2-dimensional ultrasound images in a buffer. The 3-dimensional ultrasound image forming unit 130 superposes the stored 2-dimensional ultrasound images, thereby forming a 3-dimensional ultrasound image. The selected 2-dimensional ultrasound images may be individually rendered and then stored in the buffer.

The 3-dimensional ultrasound image forming unit 130 may provide a transparent 3-dimensional ultrasound image obtained by an appropriate transparency treatment for showing a volume of interest (VOI) in the 3-dimensional ultrasound image. The 3-dimensional ultrasound image or the transparent 3-dimensional ultrasound image formed in the 3-dimensional ultrasound image forming unit 130 is transmitted to the display unit. It is then displayed on a screen of the display unit 140.

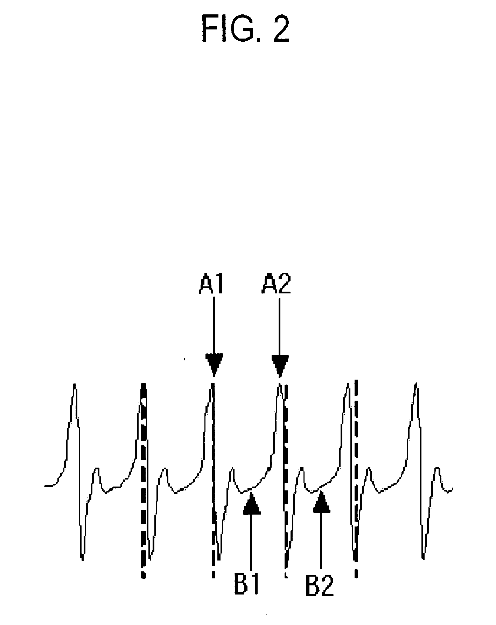

The marker setting unit 150 sets a marker representing a cycle of a heartbeat on the 3-dimensional ultrasound image. The ECG cycle setting unit 160 analyzes an ECG signal inputted from an ECG measurement device (not denoted) during a predetermined time and sets an ECG cycle. As shown in Fig. 2, for example, an interval between neighboring peaks A1 and A2 on the ECG signal may be set as an ECG cycle. Alternatively, an interval of identical phases B1 and B2, which are repeated, may be set as an ECG cycle. Information of the ECG cycle set in the ECG cycle setting unit 160 is transmitted to the marker setting unit 150.

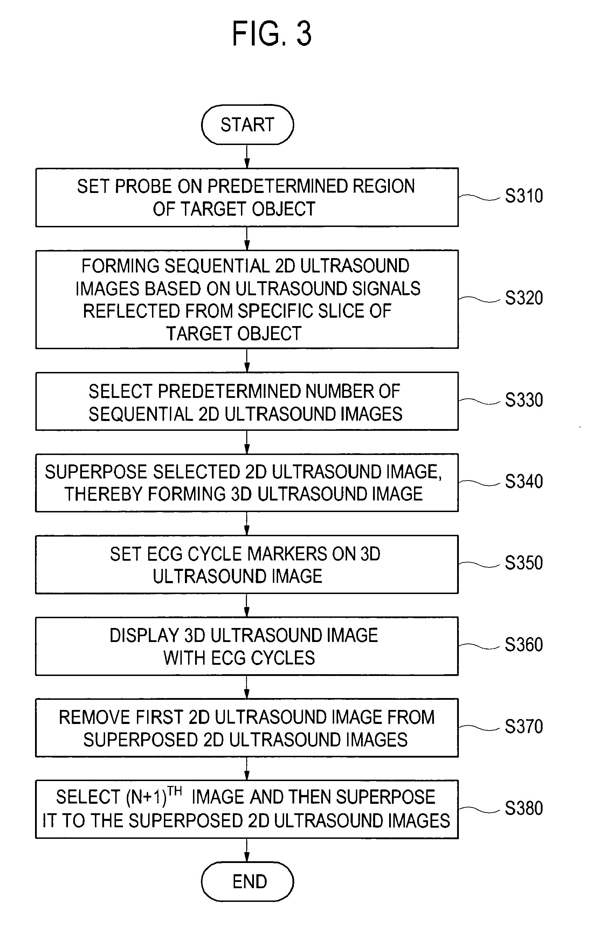

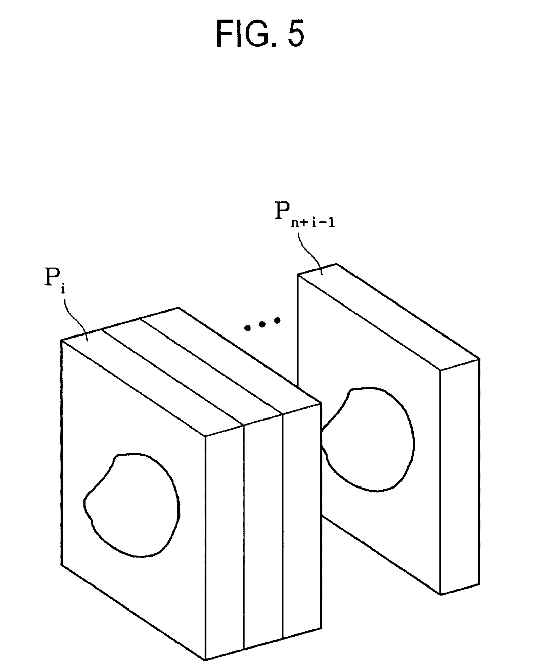

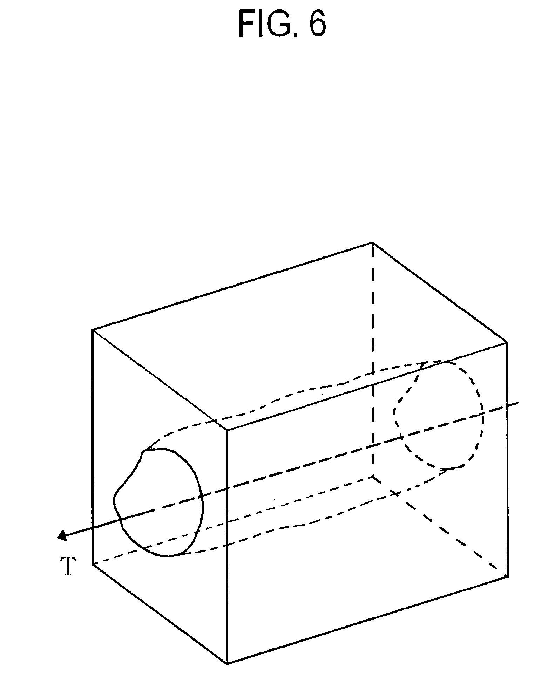

Hereinafter, a method of displaying an ultrasound image will be described in detail with reference to Figs. 3 to 8. Fig. 3 is a flowchart, which illustrates the method of displaying the ultrasound image in accordance with an embodiment of the present invention.

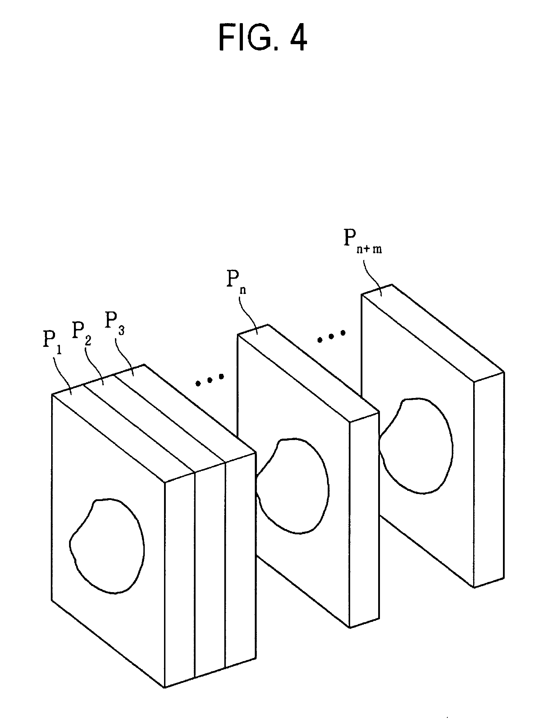

Referring to Fig. 3, the probe 110 is set on a predetermined portion of a surface of the target object to transmit ultrasound signals to a specific slice of a heart at step S310. Then, the 2-dimensional ultrasound image forming unit 120 forms a plurality of 2-dimensional ultrasound images in real time based on the ultrasound echo signals, which are reflected from the specific slice of the heart (shown in Fig. 4) at step S320. Reference symbols P₁ - Pₙ₊ₘ represent the order of forming time of the 2-dimensional ultrasound images. The 2-dimensional ultrasound images may be stored in the memory with information of the forming time.

The 3-dimensional ultrasound image forming unit 130 selects a predetermined number (e.g., n numbers) of 2-dimensional ultrasound images Pᵢ - Pₙ₊ᵢ₋₁, which are consecutive in the order of the forming time, among the multiple 2-dimensional ultrasound images. It then stores the selected 2-dimensional ultrasound images P; - Pₙ₊ᵢ₋₁ in a memory such as a buffer at step S330. The selected 2-dimensional ultrasound images may be individually rendered and then stored in the memory.

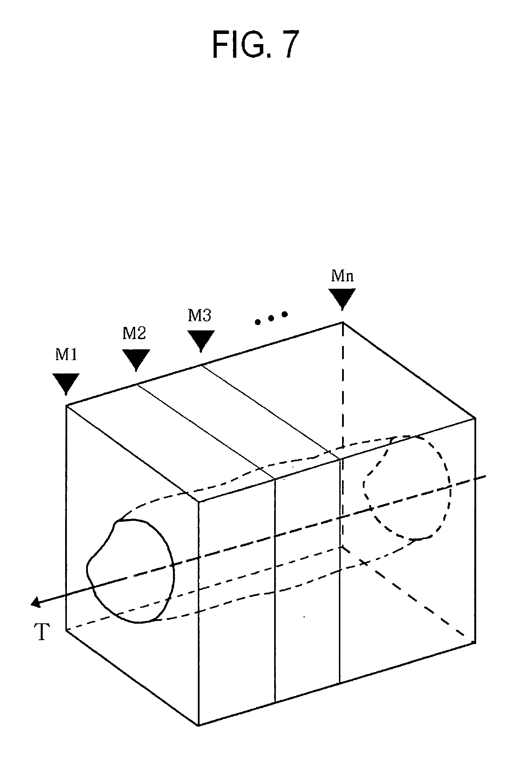

Thereafter, the stored 2-dimensional ultrasound images are superposed on a time axis, thereby forming a 3-dimensional ultrasound image at step S340. The marker setting unit 150 sets ECG cycle markers M₁ - Mₙ on the 3-dimensional ultrasound image based on the ECG cycle provided from the ECG cycle setting unit 160 at step S350. In order to set the ECG cycle markers on the 3-dimensional ultrasound image, the marker setting unit 150 selects a 2-dimensional ultrasound image Pᵢ having a foremost forming time among the 2-dimensional ultrasound images, which are used to form the 3-dimensional ultrasound image. The marker setting unit 150 then sets a reference ECG cycle marker M₁ on the selected 2-dimensional ultrasound image. The marker setting unit 150 repeatedly sets a plurality of ECG cycle markers M₂ - Mₙ by referring to the ECG cycle on the 3-dimensional ultrasound image. This is so that the 3-dimensional ultrasound image, on which the ECG cycle markers are set, is displayed on the display unit 140 (shown in Fig. 7) at step S360.

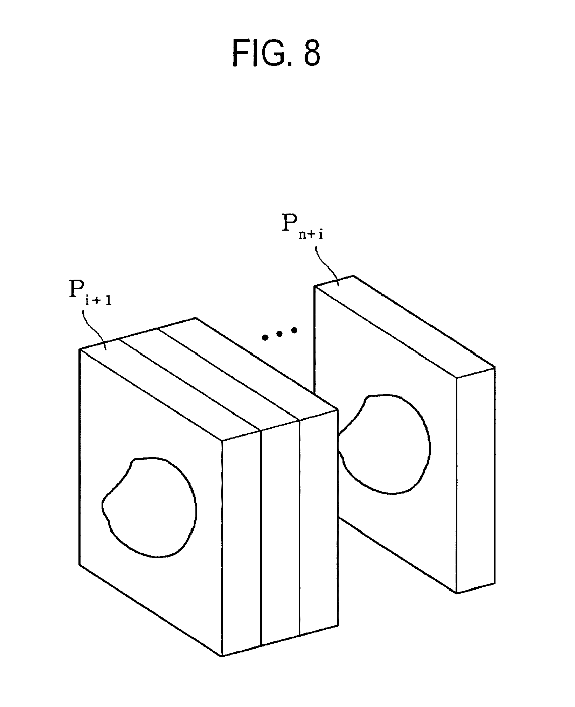

Subsequently, the 3-dimensional ultrasound image forming unit 130 removes a 2-dimensional ultrasound image Pᵢ from the 2-dimensional ultrasound images Pᵢ ~ Pᵢ₊ₙ₋₁ at step S370. The 3-dimensional ultrasound image forming unit 130 selects a 2-dimensional ultrasound image Pᵢ₊ₙ, which is next to the 2-dimensional ultrasound image Pᵢ₊ₙ₋₁, among the formed 2-dimensional ultrasound images (shown in Fig. 8) at step S380. Next, the selected 2-dimensional ultrasound image Pᵢ₊ₙ may be rendered.

Thereafter, steps S340 to S380 are repeatedly carried out for the 2-dimensional ultrasound images formed in the 2-dimensional ultrasound image forming unit 120 so as to consecutively form the 3-dimensional ultrasound images in real time. The 3-dimensional ultrasound images are displayed on the display unit 140.

An inversion mode may be applied to the 3-dimensional ultrasound image formed according to one embodiment of the present invention. This is so that the walls of blood vessels or a heart can be invisible in the 3-dimensional ultrasound image. As such, a change in the amount of blood in the blood vessel or the heart according to a time change can be easily observed. Also, the 3-dimensional ultrasound image forming unit 130 may provide a transparent 3-dimensional ultrasound image obtained by an appropriate transparency treatment for showing a volume of interest (VOI) in the 3-dimensional ultrasound image.

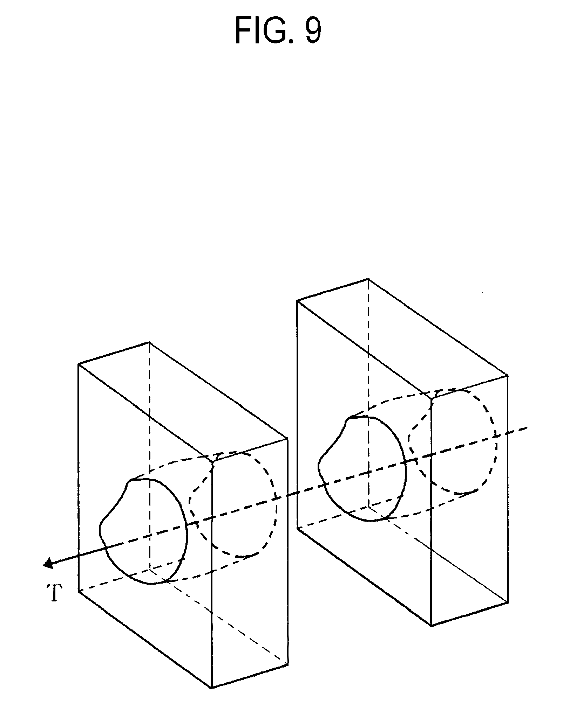

Fig. 9 is a schematic diagram showing an example of selecting two volumes, which are formed at different times in accordance with one embodiment of the present invention. Each of the selected volume is a volume obtained for one ECG cycle. Two volumes may be selected in response to a volume selection input. The selected two volumes may be compared by using arbitrary operations. For example, an operation using a set difference between two volumes may be implemented, wherein an operation result may then be displayed. Thus, the status of the heartbeat may be easily observed.

As mentioned above, the 3-dimensional ultrasound image is formed by superposing the 2-dimensional ultrasound images in accordance with one embodiment of the present invention. This is so that the 3-dimensional ultrasound image can be provided without using an expensive 3-dimensional probe. Also, as the 3-dimensional ultrasound image is formed by individually rendering the 2-dimensional ultrasound images, the required time for forming the 3-dimensional ultrasound image can be reduced. Therefore, the 3-dimensional ultrasound images may be provided in real time.

Further, since a change in the predetermined slice of a periodically moving object according to time is displayed in a 3-dimensional ultrasound image, the movement of the predetermined slice can easily observed.

In accordance with one embodiment of the present invention, there is provided a method of displaying an ultrasound image, including: a) transmitting ultrasound signals to a specific slice of a target object including a periodically moving object; b) forming and storing a plurality of sequential 2-dimensional ultrasound images based on ultrasound echo signals reflected from the specific slice, each of said sequential 2-dimensional ultrasound images being stored together with forming time information; c) selecting an n number of 2-dimensional ultrasound images in an order of forming time; d) superposing the n number of 2-dimensional ultrasound images on a time axis, thereby forming a 3-dimensional ultrasound image; e) determining a moving cycle of the moving object and setting a plurality of markers representing a moving cycle interval on the 3-dimensional ultrasound image; f) displaying the 3-dimensional ultrasound image with the moving cycle markers; g) removing a first 2-dimensional ultrasound image from the n numbers of 2-dimensional ultrasound images; h) selecting a (n+1)^{th} 2-dimensional ultrasound image referring to the forming time information from the stored 2-dimensional ultrasound images and superposing the selected 2-dimensional ultrasound image on the 2-dimensional ultrasound images with the first 2-dimensional ultrasound image removed; and i) repeating the steps e) to h) by a predetermined number of times.

In accordance with another embodiment of the present invention, there is provided an apparatus for displaying an ultrasound image, including: a probe for transmitting ultrasound signals into a specific slice of a target object including a moving object and receiving ultrasound echo signals reflected from the specific slice; a 2-dimensional ultrasound image forming unit for forming a plurality of 2-dimensional ultrasound images based on the ultrasound image signals; a storing unit for storing the plurality of 2-dimensional ultrasound images together with forming time information; a 3-dimensional ultrasound image forming unit for selecting an n number of 2-dimensional ultrasound images and superposing the n number of 2-dimensional ultrasound images on a time axis, thereby forming a 3-dimensional ultrasound image; a marker setting unit for setting at least one marker representing a moving cycle of the moving object on the 3-dimensional ultrasound image; a cycle setting unit for setting the cycle of the moving object; and a displaying unit for displaying the 3-dimensional ultrasound image and the marker.

Any reference in this specification to "one embodiment," "an embodiment," "example embodiment," etc., means that a particular feature, structure or characteristic described in connection with the embodiment is included in at least one embodiment of the invention. The appearances of such phrases in various places in the specification are not necessarily all referring to the same embodiment. Further, when a particular feature, structure or characteristic is described in connection with any embodiment, it is submitted that it is within the purview of one skilled in the art to effect such feature, structure or characteristic in connection with other ones of the embodiments.

Although embodiments have been described with reference to a number of illustrative embodiments thereof, it should be understood that numerous other modifications and embodiments can be devised by those skilled in the art that will fall within the spirit and scope of the principles of this disclosure. More particularly, various variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. A method of displaying an ultrasound image, comprising:
a) transmitting ultrasound signals to a specific slice of a target object including a periodically moving object;
b) forming and storing a plurality of sequential 2-dimensional ultrasound images based on ultrasound echo signals reflected from the specific slice, each of said sequential 2-dimensional ultrasound images being stored together with forming time information;
c) selecting an n number of 2-dimensional ultrasound images in an order of forming time;
d) superposing the n number of 2-dimensional ultrasound images on a time axis to form a 3-dimensional ultrasound image;
e) determining a moving cycle of the moving object and setting a plurality of markers representing a moving cycle interval on the 3-dimensional ultrasound image;
f) displaying the 3-dimensional ultrasound image with the moving cycle markers;
g) removing a first 2-dimensional ultrasound image from the n numbers of 2-dimensional ultrasound images;
h) selecting a (n+1)^{th} 2-dimensional ultrasound image referring to the forming time information from the stored 2-dimensional ultrasound images and superposing the selected 2-dimensional ultrasound image on the 2-dimensional ultrasound images with the first 2-dimensional ultrasound image removed; and
i) repeating the steps e) to h) by a predetermined number of times.

2. The method of Claim 1, wherein the step c) includes a step of individually rendering the selected 2-dimensional ultrasound images.

3. The method of Claim 2, wherein the 2-dimensional ultrasound image selected at step h) is rendered.

4. The method of Claim 1, wherein the moving object is a heart.

5. The method of Claim 1, wherein the moving object is a blood vessel.

6. The method of Claim 4, wherein the moving cycle is a cycle of an electrocardiogram (ECG) signal.

7. The method of Claim 6, wherein the step e) comprises:
e1) providing the ECG signal;
e2) setting the cycle of the ECG signal;
e3) setting a reference ECG cycle marker on a 2-dimensional ultrasound image having a foremost forming time among the superposed 2-dimensional ultrasound images;
e4) setting a plurality of ECG cycle markers by referring to the reference ECG cycle marker on the 3-dimensional ultrasound image.

8. The method of Claim 7, further comprising:
j) receiving a volume selection input;
k) selecting at least two volumes formed at different times and obtained for the ECG cycle from the 3-dimensional ultrasound image in response to the volume selection input; and
l) performing an operation for comparing the volumes and displaying an operation result.

9. An apparatus for displaying an ultrasound image, comprising:
a probe for transmitting ultrasound signals into a specific slice of a target object including a moving object and receiving ultrasound echo signals reflected from the specific slice;
a 2-dimensional ultrasound image forming unit for forming a plurality of 2-dimensional ultrasound images based on the ultrasound image signals;
a storing unit for storing the plurality of 2-dimensional ultrasound images together with forming time information;
a 3-dimensional ultrasound image forming unit for selecting an n number of 2-dimensional ultrasound images and superposing the n number of 2-dimensional ultrasound images on a time axis to thereby form a 3-dimensional ultrasound image;
a marker setting unit for setting at least one marker representing a moving cycle of the moving object on the 3-dimensional ultrasound image;
a cycle setting unit for setting the cycle of the moving object; and
a displaying unit for displaying the 3-dimensional ultrasound image and the marker.

10. The apparatus of Claim 9, wherein the moving object is a heart.

11. The apparatus of Claim 10, further comprising an electrocardiogram (ECG) signal providing unit, wherein the moving cycle is set with a cycle of the ECG signal.

12. The apparatus of Claim 9, wherein the selected 2-dimensional ultrasound images are individually rendered.

13. The apparatus of Claim 9, wherein the moving object is a blood vessel.
